# EUROPEAN PATENT APPLICATION

(11) **EP 2 380 571 A1**
(43) Date of publication of application: **26.10.2011**
(21) Application number: 09820301.1
(22) Date of filing: 14.10.2009
(51) Int. Cl.: A61K 31/365, C12P 17/06, C07D 309/30, C12R 1/645

(54) **BIOSYNTHESIS OF DERIVATIVES OF MONACOLIN J**

(30) Priority: 15.10.2008 ES 200802971
(71) Applicant: Neuron Biopharma, S.A., 18100 Armilla - Granada (ES)
(72) Inventor: CAMPOY GARCÍA, Sonia, E-18100 Armilla - Granada (ES); ZAFRA GÓMEZ, Alberto, E-18100 Armilla - Granada (ES); ADRIO FONDEVILA, José Luis, E-18100 Armilla - Granada (ES); VELASCO ALVAREZ, Javier, E-18100 Armilla - Granada (ES)
(74) Representative: ABG Patentes, S.L.
(86) International application number: PCT/ES2009/070436
(87) International publication number: WO 2010/043748

(57) **Abstract**

The invention relates to a process for obtaining monacolin J derivatives (I), wherein R¹ is COR², wherein R² is selected from C1-C15 alkyl, C3-C15 cycloalkyl, C2-C15 alkenyl, C2-C15 alkynyl, aryl and heterocyclyl; which comprises producing monacolin J by fermentation from a monacolin J-producing microorganism; and acylating the hydroxyl group present in the C8 position of the monacolin J previously obtained by means of adding a suitable acylating agent to the fermentation medium to obtain the desired monacolin J derivative (I).

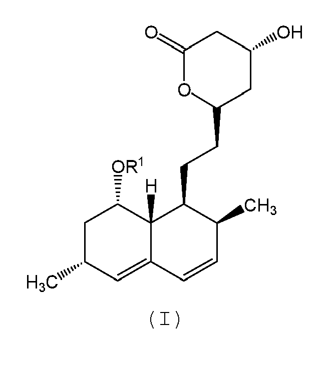

## Description

### Field of the Invention

The present invention relates to a process for obtaining monacolin J derivatives, a type of statins, which are compounds with hypocholesterolemic properties.

### Background of the Invention

Statins form a group of hypocholesterolemic agents which work by inhibiting the enzyme 3-hydroxy-3-methylglutaryl-coenzyme A (HMG-CoA) reductase which catalyzes the limiting step of cellular cholesterol biosynthesis. These compounds are used to reduce the high cholesterol levels associated with low density lipoproteins (LDL) reducing the risk of myocardial infarction and coronary death.

Natural statins (monacolin J, lovastatin, mevastatin and pravastatin) and semisynthetic statins (simvastatin) have a common polyketide structure, with a hexahydronapthalene core to which there are bound different side chains in the C8 (R¹) and C6 (R²) positions, and a lactone ring which, depending on the conditions, appears cyclized in the in the form of lactone, or open giving rise to the corresponding hydroxy acids (Formula A and Table 1).

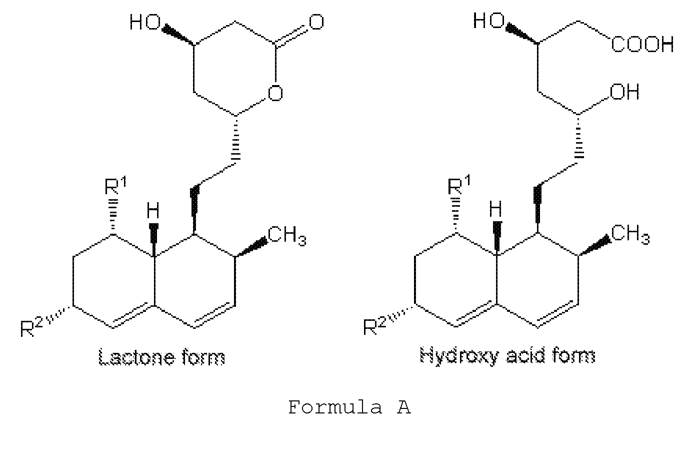

**Table 1**

| | R¹ | R² |
|---|---|---|
| Lovastatin (Monacolin K) | 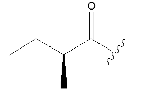 | CH₃ |
| Mevastatin | 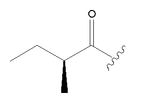 | - |
| Pravastatin | 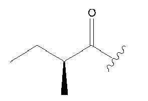 | OH |
| Simvastatin | 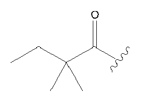 | CH₃ |
| Mevinolin (Monacolin J) | OH | CH₃ |

The biosynthesis pathways of lovastatin in *Aspergillus terreus* and of mevastatin in *Penicillium citrinum* have both been described from the biochemical point of view (Moore et al., J Am Chem Soc, 1985, 107, 3694-3701; Endo et al., J Antibiot, 1985, 38, 444-448) and on a molecular level (Hendrickson et al., Chem Biol, 1999, 6, 429-439; Kennedy et al., Science, 1999, 284, 1368-1372), and two type I polyketide synthases and several enzymes are involved. Specifically, in the case of lovastatin, lovastatin nonaketide synthase (LNS) encoded by the *LovB* gene, enoyl reductase and cytochrome P450 oxygenases give rise to monacolin J (Formula A, R¹=OH, R²=CH₃). This intermediate does not accumulate but rather directly incorporates a side chain by means of the activity of lovastatin diketide synthase (LDS) encoded by the *LovF* gene and an acetyl transferase (*LovD*).

In addition to *A. terreus* and *P. citrinum,* other microorganisms capable of producing statins (lovastatin or mevastatin) such as some species of the genera *Monascus, Doratomyces, Eupenicillium, Gymnoascus, Hypomyces, Paecylmyces, Phoma, Trichoderma, Pleurotus,* and yeasts such as *Pichia labacensis* or *Candida cariosilognicola* (US 6,943,017) are known.

Monacolin J can be obtained from culture broths of lovastatin-producing species belonging to the genus *Monascus* (JP 55139396), or also by adding monacolin K to strains of fungi belonging to different genera (eg. *Mortierella, Emericella, Humicola,* etc.) which hydrolyze the side chain giving rise to this intermediate (JP 60176595). Another strategy consists of cloning and expressing a fragment which contains the genes of *A. terreus* necessary for synthesizing monacolin J in a non lovastatin-producing strain (US 6,943,017). However, in all these cases, the yields of monacolin J are low therefore its production is not cost-effective.

Simvastatin is a semisynthetic analog of lovastatin that is more effective in the treatment of the hypercholesterolemia due to the chemical substitution of the α-methylbutyrate side chain in the C8 position (Formula A, R¹) with an α-dimethyl butyrate side chain. There are a number of chemical processes for performing this modification which include steps of hydrolysis, lactonization, protection by means of silylating and acylating monacolin J protected with α-dimethyl butyryl chloride (CA 1,199,322; Hoffman et al., J Med Chem, 1986, 29, 849-852), although the total yield is less than 40%. Variations on this process have been described in patents US 4,444,784, US 5,159,104 and US 4,450,171. In another process, lovastatin is reacted with an amine, and the diol of the resulting amide is protected and acylated with methyl iodide and a base giving rise to a diol which is lactonized, yielding simvastatin (US 4,820,850). In an improved variation of that process, the hydroxyl groups of lovastatin are protected with phenylboronic acid (US 5,393,893). Other processes are based on obtaining new intermediates by reacting lovastatin with methoxyethylamine (WO05066150), monoalkylamides or monocycloalkylamides (US 5,763,646), or by performing an enzymatic hydrolysis of lovastatin, the lactonization thereof and subsequent steps of acylation and chemical or enzymatic hydrolysis (WO05040107).

All these chemical processes require multiple steps, and are laborious; the protection steps have low yields, and the end product has impurities in the form of the non-acylated compound. All this contributes to the price of simvastatin being five times greater than that of lovastatin.

The synthesis of simvastatin by means of a chemical-biosynthetic process from lovastatin has recently been described (Xie et al., Chem Biol, 2006, 13, 1161-1169; Xie and Tang, Appl Environ Microbiol, 2007, 73, 2054-2060; W02007139871). The process starts by chemically obtaining monacolin J and an acylated substrate (α-dimethylbutyryl-S-methyl-mercaptopropionate) which are added to resting cells, or in culture cells, of *Escherichia coli* capable of overexpressing the *LovD* gene of *A. terreus* encoding an acyltransferase. The cytoplasmic-membrane permeable acylated substrate thus binds to the monacolin J giving rise to simvastatin. However, this process has limitations due to the high cost of the substrates and reagents necessary in the steps for synthesizing monacolin J and α-dimethylbutyryl-S-methyl-mercaptopropionate, as well as the partial degradation of the acylated substrate during the process.

Based on these background documents, it is deemed necessary to develop processes which solve, from the economic and technical point of view, the drawback of using raw materials which involve a higher price, and which were compatible with the environment by dispensing with or minimizing the use of chemical reagents or solvents.

### Summary of the Invention

The authors of the present invention have developed a new process for the production of monacolin J derivatives, such as simvastatin, etc., from a process that is simple, economical and favorable from the environmental point of view. Specifically, the process comprises obtaining monacolin J by fermenting and acylating the hydroxyl group present in the C8 position of monacolin J by means of adding acylating agents, which act as precursors of the side chain present in said C8 position in the monacolin J derivative, to the fermentation medium.

Therefore, in one aspect, the present invention relates to a process for obtaining a monacolin J derivative of formula (I) [defined below] which comprises producing monacolin J by fermentation from a monacolin J-producing microorganism followed by acylating the hydroxyl group present in the C8 position of the monacolin J by means of adding a suitable acylating agent to the fermentation medium to obtain the desired monacolin J derivative of formula (I).

In another aspect, the invention relates to a microorganism of the genus *Neosartorya* which has the capacity for producing and accumulating monacolin J at a concentration equal to or greater than 50 mg/L. In a particular embodiment, said microorganism is a microorganism of the species *N. stramenia.* In a specific embodiment, said microorganism is a microorganism of the species *Neosartorya stramenia* deposited in the Spanish Type Culture Collection (CECT) with accession number CECT 20472 which has the capacity for producing and accumulating monacolin J at a concentration equal to or greater than 50 mg/L, or a mutant of said microorganism that maintains the capacity for producing and accumulating monacolin J at a concentration equal to or greater than 50 mg/L. The use of said microorganism for producing monacolin J and said monacolin J derivatives of formula (I) are an additional aspect of this invention.

In another aspect, the invention relates to a biologically pure culture of said microorganism.

In another aspect, the invention relates to a process for identifying a monacolin J-producing microorganism.

In another aspect, the invention relates to a polynucleotide selected from the polynucleotides the nucleotide sequences of which are shown in SEQ ID NO: 1 and SEQ ID NO: 2. The use of said polynucleotide as a probe, or for designing primers or probes to identify strains of *Neosartorya stramenia* or other species of the genus *Neosartorya,* is an additional aspect of this invention.

### Detailed Description of the Invention

### Definitions

The present invention relates to the production of monacolin J derivatives of formula (I) as defined below. In said compounds of formula (I), the meanings indicated below are understood.

The term "alkyl" refers to a radical of a linear or branched hydrocarbon chain consisting of carbon and hydrogen atoms, which is not unsaturated and is bound to the rest of the molecule by a single bond, for example, methyl, ethyl, propyl, isopropyl, butyl, *sec*-butyl, *tert*-butyl, etc. The alkyl group can be optionally substituted.

The term "cycloalkyl" refers to a stable monocyclic or bicyclic radical which is saturated or partially saturated, consisting of carbon and hydrogen atoms, such as cyclopropyl, cyclobutyl, cyclopentyl, cyclohexyl, adamantyl, etc. The cycloalkyl group can be optionally substituted.

The term "alkenyl" refers to an optionally substituted radical of a linear or branched hydrocarbon chain having one or more carbon-carbon double bonds and which is bound to the rest of the molecule by a single bond, for example, vinyl, allyl, etc. The alkenyl group can be optionally substituted.

The term "alkynyl" refers to an optionally substituted radical of a linear or branched hydrocarbon chain having one or more carbon-carbon triple bonds and which is bound to the rest of the molecule by a single bond, for example, ethynyl, 1-propynyl, etc. The alkynyl group can be optionally substituted.

The term "aryl" refers to a radical of an aromatic hydrocarbon containing one or several rings, including multiple rings with separated or fused aryl radicals; typical aryl groups contain 1 to 3 separated or condensed rings and from 6 to approximately 18 carbon atoms, for example, phenyl, naphthyl, indenyl, phenanthryl, anthracyl, etc. The aryl groups can be optionally substituted.

The term "heterocyclyl" refers to a stable radical of a 3 to 15 member ring containing carbon atoms and from one to five heteroatoms selected from the group consisting of nitrogen, oxygen and sulfur, preferably a 4 to 8 member ring with one or more heteroatoms, more preferably a 5 or 6 member ring with one or more heteroatoms. For the purpose of this invention, the heterocycle can be a monocyclic, bicyclic or tricyclic ring system which may include condensed ring systems and the nitrogen, carbon or sulfur atom in the heterocyclyl radical can be optionally oxidized, the nitrogen atom can be optionally quaternized, and the heterocyclyl radical can be partially or completely saturated or it can be aromatic. Non-limiting illustrative examples of such heterocyclyl radicals include pyridyl, pyrazinyl, pyrimidyl, furyl, pyrrolyl, thienyl, thiazolyl, thiadiazolyl, oxazolyl, imidazolyl, indolyl, isoxazolyl, benzofuranyl, benzothiazolyl, benzimidazolyl, isothiazolyl, piperidyl, quinolyl, tetrahydrofuranyl, tetrahydropyranyl, piperidinyl, morpholino, pyrrolidinyl, etc. The heterocyclyl groups can be optionally substituted.

As indicated, the aforementioned groups can be optionally substituted in one or several of their available positions independently with one or several suitable substituents, such as OR', =O, SR', SOR', SO₂R', NO₂, NHR', N(R')₂, =N-R', NHCOR', N(COR')₂, NHSO₂R', CN, halogen, C(=OR)R', COOR', OC(=O)R', substituted or non-substituted aryl and substituted or non-substituted heterocycle, wherein R' is selected independently from H, OH, NO₂, NH₂, SH, CN, halogen, C(=O)H, C(=O)CH₃, COOH, substituted or non-substituted C1-C12 alkyl, substituted or non-substituted C2-C12 alkenyl, substituted or non-substituted C2-C12 alkynyl and substituted or non-substituted aryl. The halogen substituents that may be present in the compounds of formula (I) include F, Cl, Br and I.

Functional groups such as hydroxyl or amino can be optionally protected. There is a large number of protecting groups for different functional groups, such as hydroxyl and amino, and they are well known by the person skilled in the art. As a guide, see "Protecting groups", Kocienski, 2004, 3rd edition and Greene and Wuts "Protective Groups in Organic Synthesis", John Wiley & Sons, Inc., New York, 1999.

The term "lower alcohol" refers to a compound comprising an -OH group and 1 to 8 carbon atoms.

### Producing monacolin J derivatives

In one aspect, the present invention relates to a process, hereinafter the process of the invention, for obtaining a monacolin J derivative of formula (I) 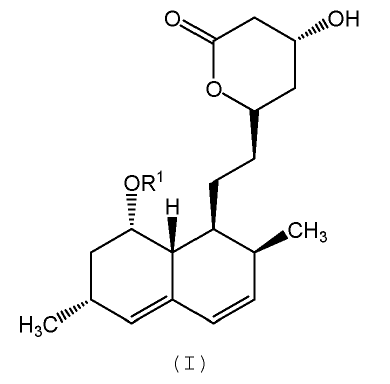 wherein R¹ is COR², wherein R² is selected from C1-C15 alkyl, C3-C15 cycloalkyl, C2-C15 alkenyl, C2-C15 alkynyl, aryl and heterocyclyl;
which comprises the steps of:
a) producing monacolin J by fermentation from a monacolin J-producing microorganism; and
b) acylating the hydroxyl group present in the C8 position of the monacolin J obtained in step a) by means of adding a suitable acylating agent to the fermentation medium to obtain the desired monacolin J derivative of formula (I).

In the first step [step a)], the process of the invention comprises producing monacolin J by fermentation from a monacolin J-producing microorganism. Although virtually any monacolin J-producing microorganism can be used to produce the monacolin J derivative of formula (I) according to the process of the invention, from the point of view of its industrial application, the monacolin J-producing microorganism used in step a) is capable of producing a large amount of monacolin J; advantageously in addition to producing a large amount of monacolin J, said monacolin J-producing microorganism must not produce monacolin J derivatives with the hydroxyl group present in the acylated C8 position, e.g., lovastatin, etc., or, in the event that it does produce them, they must be produced in very small amounts. In this sense, if said monacolin J-producing microorganism produced, in addition to monacolin J, other monacolin J derivatives, for example, lovastatin, etc., the production of monacolin J must advantageously be greater than the production of said monacolin J derivative; by way of illustration, if the monacolin J-producing microorganism also produces lovastatin, the production of monacolin J will preferably be at least 10 times greater than the production of lovastatin.

In a particular embodiment, the monacolin J-producing microorganism used for producing the compound of formula (I) according to the process of the invention is a microorganism capable of producing and accumulating monacolin J at a concentration equal to or greater than 50 mg/L, advantageously equal to or greater than 100 mg/L, preferably equal to or greater than 250 mg/L, more preferably equal to or greater than 500 mg/L, yet more preferably equal to or greater than 750 mg/L, and still more preferably equal to or greater than 1,000 mg/L of culture broth. Non-limiting illustrative examples of monacolin J-producing microorganisms susceptible to being used in the process of the invention include strains belonging to the genera *Aspergillus, Monascus, Penicillium,* and, now, *Neosartorya.* In a specific embodiment, the monacolin J-producing microorganism used for producing the monacolin J derivative of formula (I) according to the process of the invention is a filamentous fungi belonging to the genus *Neosartorya,* such as a fungi of the species *N. stramenia.* In a preferred embodiment, said monacolin J-producing microorganism used for producing the monacolin J derivative of formula (I) according to the process of the invention, is a strain of *Neosartorya stramenia* deposited in the Spanish Type Culture Collection (CECT) with accession number CECT 20472, which has the capacity for producing and accumulating monacolin J at a concentration equal to or greater than 50 mg/L, or a mutant of said microorganism that maintains the capacity for producing and accumulating monacolin J at a concentration equal to or greater than 50 mg/L, the characteristics of which will be described in detail below, occasionally identified in this description as "microorganism of the invention".

For producing monacolin J by fermentation, the monacolin J-producing microorganism will be cultured under suitable conditions which allow the production of monacolin J. Generally, said conditions, such as the culture medium, the carbon source, the nitrogen source, the temperature, etc., will be selected depending on the nature of the monacolin J-producing microorganism selected.

Non-limiting illustrative examples of carbon sources include glucose, maltose, sucrose, mannitol, glycerol, molasses, polyethylene glycol, starch, fatty acids, oils, etc. Likewise, non-limiting illustrative examples of nitrogen sources include both organic nitrogen sources such as yeast extract, peptone, corn steep liquor, urea, peptonized milk, sodium glutamate, etc., and inorganic nitrogen sources such as different ammonium salts, etc.

In a particular embodiment, the culture of the monacolin J-producing microorganism is performed for a time period comprised between 5 and 15 days at a temperature comprised between 20°C and 35°C, preferably between 28°C and 32°C depending on the microorganism, with constant stirring, generally under aerobic conditions.

The monacolin J produced by the monacolin J-producing microorganism can be in the form of lactone (more stable), in the form of hydroxy acid (more abundant) or in both forms as a mixture of the closed form (lactone) and open form (hydroxy acid) form.

Then, once the desired amount of monacolin J, for example, the maximum amount of monacolin J, is reached, a suitable acylating agent is the added to the culture medium for the purpose of acylating the hydroxyl group present in the C8 position of the monacolin J and obtaining the desired monacolin J derivative of formula (I) [step b)].

According to the invention, the chemical acylation of monacolin J consists of transforming the hydroxyl present in the C8 position of monacolin J into an ester. This transformation, which gives rise to the formation of different side chains in the C8 position of monacolin J, is achieved by means of adding the suitable acylating agent to the culture medium. Without wishing to be bound by any theory, it is believed that said acylation reaction occurs inside the cell, probably with the collaboration of an enzyme, such as an acyltransferase encoded by the lovD gene or an ortholog thereof, therefore in a particular embodiment, the monacolin J-producing microorganism used for producing the monacolin J derivative of formula (I) according to the process of the invention encodes said acyltransferase, either in the native form or recombinant form.

Although the most common acylating agents are carboxylic acids and derivatives thereof, such as the halides (particularly, chlorides), esters, amides, anhydrides or salts thereof, any suitable compound capable of acylating the hydroxyl group present in the C8 position of monacolin J and forming an ester in said C8 position of monacolin J can be used as an acylating agent in the present invention for obtaining the desired monacolin J derivative of formula (I). Said acylating agents can be easily identified by the person skilled in the art. However, in a particular embodiment, said acylating agent is a compound of formula (II)

R²COOH (II)

wherein R² has the meaning previously indicated in relation to formula (I); or
a derivative thereof selected from a halide, an ester, an amide, an anhydride or a salt of said carboxylic acid of formula (II).

The compounds of formula (II), e.g., propanoic acid, 2,2-dimethylpropanoic acid, 2-methylbutanoic acid, 2,2-dimethylbutanoic acid, etc., are known compounds or can be obtained by conventional methods known by persons skilled in the art.

The halides of the carboxylic acid of formula (II) can be obtained by conventional methods, for example, by reacting said carboxylic acid with SOCl₂, PCl₅, PBr₃, ClCOCOCl, etc. The esters of the carboxylic acid of formula (II) can be easily obtained by conventional methods, for example, by reacting said carboxylic acid, or an anhydride or chloride thereof, with the corresponding alcohol, or by reacting the sodium salt of said carboxylic acid (II) with an alkyl halide, etc. The amides of the carboxylic acid of formula (II) can also be easily obtained by conventional methods, for example, by reacting an ester, an anhydride or a halide of said carboxylic acid with ammonia or with an amine, or by means of hydrolyzing the corresponding nitryl, etc. The anhydrides of the carboxylic acid of formula (II) can be easily obtained by conventional methods, for example, by reacting a halide of said carboxylic acid with a carboxylate, etc. The salts of the carboxylic acid of formula (II) include metal salts, e.g., sodium salt, potassium salt, ammonium salt, etc., which can be easily obtained by conventional methods, for example, by reacting said carboxylic acid with the suitable base.

Non-limiting illustrative examples of said acylating agents include acetates, propionates, such as sodium propionate, sodium 2,2-dimethylpropionate, etc., butyrates, such as 2-methylbutyrate, 2,2-dimethylbutyrate, etc. If chiral centers are present, the acylating agents will preferably be in the desired enantiopure form, for example, sodium (*S*)-2-methylbutyrate, etc.

Non-limiting illustrative examples of monacolin J derivatives of formula (I) obtained according to the process of the invention include those compounds of formula (I) wherein R¹ is selected from propionyl, 2,2-dimethylpropionyl, 2-methylbutyryl (lovastatin) and 2,2-dimethylbutyryl (simvastatin).

In a particular embodiment, the acylation reaction is carried out with stirring for a time period comprised between 24 and 72 hours, at a temperature comprised between 20°C and 40°C, preferably between 25°C and 30°C.

The monacolin J derivative of formula (I) obtained, if desired, can be isolated and purified by conventional methods. To that end, said compound of formula (I) can be extracted from the culture broth and, if desired, concentrated and optionally crystallized.

The purpose of extracting the compound of formula (I) obtained is to separate it from the rest of the compounds present in the culture broth (fermentation medium). Said compound of formula (I) can be extracted from the culture by conventional methods, for example, by extraction with a suitable solvent in acidic medium. To that end, the culture broth containing the compound of formula (I) is acidified by means of using a suitable organic or inorganic acid, typically inorganic acid. In a particular embodiment, said culture medium is acidified to a pH value comprised between 2.5 and 5, preferably between 3 and 4. Non-limiting illustrative examples of said acids which can be used for acidifying said culture medium include any acid capable of acidifying said culture medium up to a suitable pH value, for example, between 2.5 and 5, for example, hydrochloric acid, sulfuric acid, phosphoric acid, etc.

In a particular embodiment, said extraction can be performed using a suitable solvent, such as an organic solvent, for example, an ester, such as methyl acetate, ethyl acetate, propyl acetate, butyl acetate, etc. The amount of solvent to be added may vary within a broad range; however, in a particular embodiment, said solvent is added in an amount comprised between 0.5 and 2 times the culture volume. The extraction can be carried out, if desired, with stirring for a time period comprised between 1 and 2 hours at controlled speed. The resulting phases can be subsequently separated by conventional methods, for example, by decantation or centrifugation.

Once separated, the isolated compound of formula (I), which can be in a closed form (lactone), an open form (hydroxy acid) or a in a mixed form, i.e., in a mixture of said closed and open forms, if desired, can be concentrated by conventional methods, for example, by means of simultaneous lactonization of the hydroxy acid form with a vacuum and subsequent crystallization, for example, by means of cooling at a temperature comprised between -20°C and -30°C. If desired, the compound of formula (I) can additionally be subjected to a recrystallization step for the purpose of increasing its purity. In a particular embodiment, the previously obtained crystals of the compound of formula (I) are filtered and dried under vacuum at a temperature comprised between 40°C and 60°C, preferably between 45°C and 50°C. Said crystals can be solubilized by adding a suitable solvent, for example, an ester, such as a lower alcohol acetate, for example, methyl acetate, ethyl acetate, propyl acetate or butyl acetate, and are crystallized by cooling at a temperature comprised between -20°C and -30°C, as described previously.

### Microorganism of the invention

In one aspect, the invention relates to a microorganism, hereinafter microorganism of the invention, of the genus *Neosartorya,* which has the capacity for producing and accumulating monacolin J at a concentration equal to or greater than 50 mg/L. In a particular embodiment, said microorganism is a microorganism of the species *N. stramenia.* In a specific embodiment, said microorganism of the invention is a microorganism of the species *Neosartorya stramenia* deposited in the Spanish Type Culture Collection (CECT) with accession number CECT 20472 which has the capacity for producing and accumulating monacolin J at a concentration equal to or greater than 50 mg/L, or a mutant of said microorganism that maintains the capacity for producing and accumulating monacolin J at a concentration equal to or greater than 50 mg/L. As it is used herein, the expression "capacity for producing and accumulating monacolin J at a concentration equal to or greater than 50 mg/L", applied to a microorganism, means that under suitable conditions, said microorganism is capable of producing monacolin J reaching a concentration greater than 50 milligrams (mg) of monacolin J per liter (L) of culture broth. Said suitable conditions involve culturing in a suitable culture medium and at a suitable temperature. In a particular embodiment, the microorganism of the invention is capable of producing and accumulating monacolin J at a concentration equal to or greater than 50 mg/L, advantageously equal to or greater than 100 mg/L, preferably equal to or greater than 250 mg/L, more preferably equal to or greater than 500 mg/L, yet more preferably equal to or greater than 750 mg/L, and still more preferably equal to or greater than 1,000 mg/L.

The capacity of a microorganism for producing and accumulating monacolin J (e.g., at a concentration equal to or greater than 50 mg/L) can be determined by any conventional process, for example, by inoculating a culture of said microorganism in a suitable culture medium, incubating under suitable conditions and measuring the amount of monacolin J produced, as described, for example, in Example 1 included in the present description. In a particular embodiment, the microorganism of the invention is the strain *N. stramenia* CECT 20472.

In another particular embodiment, the microorganism of the invention is a mutant of said strain *N. stramenia* CECT 20472 that maintains the capacity for producing and accumulating monacolin J at a concentration equal to or greater than 50 mg/L. As it is used herein, the term "mutant "includes any individual or organism resulting from a mutation or change in the DNA of a gene of an organism resulting in a character (phenotype) that is not found in the wild type and any individual or organism resulting from a mutation in the DNA of a gene of an organism that does not produce a detectable phenotypic effect (silent mutation); non-limiting illustrative examples of said mutations or changes in the DNA include the insertion or deletion of nucleotides as well as the substitution of some nucleotides with other nucleotides; in a specific embodiment, said mutant is a mutant of *N. stramenia* CECT 20472 which essentially maintains the same characteristics as those of the parental strain [*N. stramenia* CECT 20472], and further has the capacity for not producing, or producing very small amounts of, lovastatin. The mutants can be obtained by means of conventional techniques known by persons skilled in the art, such as classic or directed mutagenesis, genetic manipulation, recombination, etc.

The microorganism of the invention can be used for producing monacolin J by means of a microbiological process (fermentation) and producing a monacolin J derivative of formula (I), e.g., simvastatin, lovastatin, etc., from said compound. From the point of view of the industrial application of the process of the invention, the monacolin J-producing microorganism is preferably capable of producing a large amount of monacolin J, and does not produce, or produces in minor amounts, other monacolin J derivatives, e.g., lovastatin. In a preferred embodiment, the microorganism of the invention is capable of producing monacolin J in an amount equal to or greater than 10 times the amount of lovastatin produced (if said compound is produced).

The microorganism of the invention has been isolated from a screening for monacolin J-producing microorganisms. To that end, briefly, blocks of cultures of different microorganisms grown in plates were extracted and deposited on other plates previously inoculated with a culture of *Candida albicans* and, after incubation, the existence of fungicidal activity was determined by means of the formation of *C. albicans* growth inhibition halos, selecting among the strains which showed fungicidal activity those that showed *C. albicans* growth inhibition halos with a size smaller than that produced by the strain *Aspergillus* terreus ATCC 20542 used as control. Subsequently, the monacolin derivatives were extracted and analyzed by UPLC-PDA-MS/MS against the pure patterns of lovastatin, mevastatin and monacolin J, as indicated in Example 1. Following this process, a strain was isolated which produced mainly monacolin J and a small amount of lovastatin, which was identified, by means of sequencing the D1/D2 region of 28S subunit of the ribosomal DNA (rDNA) (SEQ ID NO: 1) and a fragment of the complete ITS (Internal Transcribed Spacer) region located between the 18S and 28S subunits (SEQ ID NO: 2), as *Neosartorya stramenia* and deposited in the Spanish Type Culture Collection(CECT) with accession number CECT 20742.

Said polynucleotides, the nucleotide sequences of which are shown in SEQ ID NO: 1 and SEQ ID NO: 2, can be used as probes or for designing primers or probes therefrom, for identifying other strains of *N. stramenia* or other species of the genus *Neosartorya;* therefore said polynucleotides and their applications are additional aspects of the present invention.

The D1/D2 region of 28S subunit of the rDNA (SEQ ID NO: 1) of *N. stramenia* CECT 20742 can be amplified by means of polymerase chain reaction (PCR) using universal fungal oligonucleotide primers the nucleotide sequences of which are shown in SEQ ID NO: 3 and SEQ ID NO: 4. The complete ITS region located between the 18S and 28S subunits (SEQ ID NO: 2) can also be amplified by means of PCR using the universal fungal oligonucleotide primers the nucleotide sequences of which 1 are shown in SEQ ID NO: 5 and SEQ ID NO: 6.

A biologically pure culture of a microorganism of the invention is an additional aspect of the present invention.

As previously mentioned, the microorganism of the invention can be used for producing monacolin J by means of a microbiological process (fermentation) and producing a monacolin J derivative of formula (I), e.g., simvastatin, lovastatin, etc., from said compound. Therefore, in another aspect, the invention relates to the use of said microorganism of the invention for producing monacolin J or a monacolin J derivative of formula (I). In a particular embodiment, the microorganism of the invention is a fungus of the species *N. stramenia;* in a specific embodiment, said microorganism of the invention is the strain *N. stramenia* CECT 20472.

### Process for identifying monacolin J-producing microorganisms

In another aspect, the invention relates to a process for identifying a monacolin J-producing microorganism (as a product of biosynthesis), which comprises:
a) incubating a culture of a microorganism in a plate inoculated with a culture of *Candida albicans* under conditions which allow the growth of said strain and of *C. albicans;*
b) analyzing the existence of antifungal activity associated with said microorganism;
c) if said microorganism does not show antifungal activity or shows low antifungal activity, collecting a sample from the culture of said microorganism and analyzing it to detect and/or quantify monacolin J in said sample; and
d) if said analysis shows the presence of monacolin J, identifying said microorganism as a monacolin J-producing microorganism.

The process comprises contacting a culture of a microorganism with a culture of *C. albicans* deposited on a plate under conditions which allow the growth of said strain and of *C. albicans.* Said conditions are known by persons skilled in the art; nevertheless, in a particular embodiment, a culture of the microorganism to be assayed is deposited on plates with a medium containing malt extract, glucose, mycopeptone and agar previously inoculated with a culture of *C. albicans* (e.g., *C. albicans* CECT 1002); the plates are then maintained at 4°C for 1 hour and are subsequently incubated at 28°C for one night.

The existence of antifungal activity associated with said microorganism is then analyzed by any suitable conventional method for the purpose of selecting those microorganisms with nil or low antifungal activity; however, in a particular embodiment, the existence of antifungal activity associated with said microorganism is analyzed by means of the formation of *C. albicans* growth inhibition halos. To evaluate if a microorganism does not show antifungal activity or shows low antifungal activity, it may be appropriate to compare said eventual antifungal activity with the antifungal activity of a positive control and those microorganisms showing lower antifungal activity than the control or those that do not show antifungal activity are selected. Therefore, in a particular embodiment, a positive control for antifungal activity that is inoculated on a plate inoculated with *C. albicans* under suitable conditions which allow the growth of both said control microorganism as well as *C. albicans* is used; although virtually any antifungal compound-producing microorganism can be used, it is interesting in practice to use statin- (e.g., lovastatin) producing microorganisms since the β-hydroxy acid form of lovastatin has antifungal properties, and to select microorganisms that show inhibition halos with a size smaller than that produced by said control; in a specific embodiment, the strain *Aspergillus* terreus ATCC 20542 (Example 1) is used as control.

If the microorganism in question shows virtually nil or low antifungal activity, a sample is collected from the culture of said microorganism culture and analyzed to detect and/or to quantify monacolin J in said sample. Virtually any analysis suitable for detecting or quantifying monacolin J can be used; however, in a particular embodiment, the detection and quantification of monacolin J is carried out by means of Ultra Performance Liquid Chromatography-Mass Spectrometry/Mass Spectrometry (UPLC-PDA-MS/MS) against pure patterns of monacolin J. Other related compounds, e.g., lovastatin or mevastatin (Example 1), can be additionally or optionally analyzed. To that end, monacolin J is first extracted by means of conventional methods, for example, by means of extraction with a suitable solvent (e.g., an ester, such as ethyl acetate, etc.) in acidic medium (e.g., HCl, etc.) as described in Example 1.

In a particular embodiment, the monacolin J-producing microorganism is a microorganism capable of producing and accumulating monacolin J at a concentration equal to or greater than 50 mg/L, advantageously equal to or greater than 100 mg/L, preferably equal to or greater than 250 mg/L, more preferably equal to or greater than 500 mg/L, yet more preferably equal to or greater than 750 mg/L, and still more preferably equal to or greater than 1,000 mg/L.

If said analysis shows the presence of monacolin J, said microorganism is identified as a monacolin J-producing microorganism. If necessary, said monacolin J-producing microorganism is characterized by suitable methods depending on its nature, e.g., by means of classic microbiological methods, analyzing the specific regions of the genome of the genus, species or strain (e.g., specific regions of the 28S subunit of the ribosomal DNA, specific ITS regions, etc.) by means of using conventional techniques, e.g., polymerase chain reaction (PCR) amplification, etc.

Non-limiting illustrative examples of monacolin J-producing microorganisms identified according to the process provided by this invention include strains belonging to the genera *Aspergillus, Monascus, Penicillium,* and, now, *Neosartorya.* In a particular embodiment, the monacolin J-producing microorganism identified according to said process is the strain *N. stramenia* CECT 20472 which has the capacity for producing and accumulating monacolin J at a concentration equal to or greater than 50 mg/L.

The following examples illustrate the invention and must not be considered as limiting thereof.

### EXAMPLE 1

### Selection of microorganisms capable of producing and accumulating monacolin J

Blocks 0.6 cm in diameter were extracted from cultures of different fungi isolated from soil samples grown in plates with M2 medium (containing per liter: 45 g of glucose, 2.5 g of polyethylene glycol P2000, 24 g of peptonized milk and 2.5 g of yeast extract) and were deposited on plates with MA medium (containing per liter: 20 g of malt extract, 20 g of glucose, 1 g of mycopeptone and 10 g of agar) previously inoculated with a culture of *Candida albicans* CECT 1002. The plates were maintained at 4°C for 1 hour and were subsequently incubated at 28°C overnight. The existence of antifungal activity was determined by means of the formation of *C. albicans* growth inhibition halos. Those strains that showed inhibition halos with a size smaller than that produced by the strain *Aspergillus* terreus ATCC 20542 (lovastatin-producing strain) used as control were selected from the strains which showed fungicidal activity.

Blocks 0.6 cm in diameter, which were introduced in a 2 mL Eppendorf tube, were extracted from the plates of the selected strains; 1 mL of acidified ethyl acetate (acidified with HCl) was then added and the tube was placed in an ultrasonic bath for 10 minutes. It was then centrifuged at 12,000 rpm for 3 minutes and the supernatant was separated into a new tube and dried under nitrogen flow. The precipitate was resuspended in 1 mL of methanol, filtered and analyzed by UPLC-PDA-MS/MS against pure patterns of lovastatin, mevastatin and monacolin J.

By means of this process, 197 strains of different microorganisms capable of producing compounds with fungicidal activity were isolated, 186 of which showed inhibition halos with a size smaller than that of strain *A. terreus* ATCC 20542 (control). The UPLC-PDA-MS/MS analysis allowed identifying 3 strains which produced lovastatin, 2 strains which produced mevastatin and 1 strain which produced mainly monacolin J and a small amount of lovastatin. The latter was identified by means of sequencing the D1/D2 region of the 28S subunit of the ribosomal DNA (rDNA) and a fragment of the complete ITS (Internal Transcribed Spacer) region located between the 18S and 28S subunits, as *Neosartorya stramenia,* which did not bear any similarity to the sequences of this species deposited in the existing databases. The strain *Neosartorya stramenia* has been deposited in the Spanish Type Culture Collection (CECT), Burjassot, Valencia (Spain) on 16 January 2008 with accession number CECT 20742.

The amplification of the D1/D2 region of the 28S subunit of the rDNA of said microorganism was carried out by means of polymerase chain reaction (PCR), using the oligonucleotide primers the nucleotide sequences of which are shown in SEQ ID NO: 3 and SEQ ID NO: 4, respectively. The conditions for carrying out PCR were the following: (i) 96°C for 5 minutes; (ii) 30 cycles (94°C, 30 seconds; 60°C, 40 seconds; 72°C, 1 minute); and finally, (iii) an elongation cycle at 72°C for 10 minutes. The complete ITS region located between the 18S and 28S subunits was also amplified by means of PCR, using the oligonucleotide primers the nucleotide sequences of which are shown in SEQ ID NO: 5 and SEQ ID NO: 6, respectively. The conditions for carrying out the PCR were the same as those used for amplifying said D1/D2 region.

### EXAMPLE 2

### Producing monacolin J by fermentation

A suspension of spores of *N. stramenia* CECT 20742 was used to inoculate plates with Power medium (containing per liter: 15 g of sucrose, 2.5 g of bacteriological peptone, 2.5 g of lactose, 0.5 g of corn steep liquor, 2 g of NaCl, 1 g of NaNO₃, 26.1 g of KCl, 0.25 g of K₂HPO₄, 0.25 g of MgSO₄ · 7H₂O, 0.03 g of KH₂PO₄, 0.005 g of FeSO₄ · 7H₂O, 0.0015 g of FeCl₃ · 6H₂O and 0.0005 g of CUSO₄-5H₂O) and was incubated at 28°C for 5 days. The spores of each plate were collected in 5 mL of 20% glycerol and were used for inoculating flasks containing 30 mL of M2 medium (Example 1). The flasks were incubated at 28°C and 200 rpm of stirring for 48 hours.

These cultures were used to inoculate flasks containing M2 medium and were incubated at 28°C, 200 rpm for 2-5 days. The broths were then mixed, a 310 mg content of monacolin J being obtained at a concentration of 70 mg/L, determined by means of UPLC-PDA which was purified as indicated below. The broth was centrifuged at 5,000 rpm for 10 minutes and the supernatant were separated from the broth and mycelium. The latter was resuspended in 150 mL of water and was subjected to vigorous stirring for 1 hour. The process was repeated twice. The filtrates were mixed with the supernatant of the broth and the pH was adjusted to 3.5 with diluted sulfuric acid. The extraction was performed with 3 x 300 mL of ethyl acetate under constant stirring for 30 minutes each time. The combined ethyl acetate extracts were dried with anhydrous sodium sulfate and concentrated in a vacuum until obtaining a volume of 100 mL. Lactonization was then performed by means of adding trifluoroacetic acid at room temperature and with constant stirring. The formation of lactones was confirmed by means of UPLC-MS/MS. After the formation of lactones is completed, it was washed with 2 x 20 mL of 5% aqueous sodium hydrogen carbonate and then with 20 mL of water, it was dried with anhydrous sodium sulfate and evaporated in a vacuum. The residue obtained was then resuspended in 40 mL of ethyl acetate and n-hexane (20:80) and was passed through a silica gel column 1.2 cm in diameter and with a bed height of 20 cm. The elution was performed by means of mixtures of ethyl acetate and n-hexane, the concentration of ethyl acetate being gradually increased. The fractions containing the lactone of monacolin J were eluted from the column into a mixture of 45% ethyl acetate and 55% n-hexane. The fractions were combined and evaporated in a vacuum. The residue obtained was dissolved in 10 mL of acetone and was kept at 4°C for one night. The precipitate was filtered, washed with 2 mL of acetone and with 2 mL of n-hexane, being dried in a vacuum at room temperature. The monacolin J obtained was resuspended in 20 mL of methanol, bleached by adding 10 g of activated carbon and crystallized at -20°C with a mixture of ethanol-ethyl acetate. Finally, the crystals were dried in a vacuum at room temperature.

### EXAMPLE 3

### Producing simvastatin

A suspension of spores of *N. stramenia* CECT 20742 prepared as indicated in Example 2 was used for inoculating flasks containing 25 mL of MEB medium (containing per liter: 15 g of malt extract, 1 g of bacteriological peptone and 20 g of glucose). The flasks were incubated at 28°C and 250 rpm of stirring for 48 hours. These cultures were used for inoculating (10% v/v) flasks containing 50 mL of M2 medium (Example 1) and they were incubated at 28°C, 250 rpm for 48 hours. After that time, sodium 2,2-dimethylbutyrate (0.1%) (w/v) was added to the cultures maintaining the same incubation conditions. The cultures were grown for another 72-96 hours.

The presence of simvastatin in the culture broth was checked by means of taking samples from the broth, which were extracted with ethyl acetate, concentrated in a vacuum and resuspended in methanol before analyzing them by means of UPLC. Finally, the culture broth was subjected to the process of washing, extraction and purification indicated in Example 2.

### EXAMPLE 4

### Producing the compound of formula (I) wherein R¹ is propionyl

Flasks containing 50 mL of M2 medium were inoculated (10% v/v) with inocula grown in MEB medium as indicated in Example 3 and they were incubated at 28°C, 250 rpm for 48 hours. After that time, sodium propionate (0.1%) (w/v) was added to the cultures maintaining the same incubation conditions. The cultures were grown for another 72-96 hours.

The presence of the compound of formula (I) wherein R¹ is propionyl, [(1*S*,3*R*,7*R*,8*S*,8*aR*)-8-[2-[(2*R*,4*R*)-4-hydroxy-6-oxo-oxan-2-yl]ethyl]-3,7-dimethyl-1,2,3,7,8,8*a*-hexahydro-naphthalen-1-yl]propionate in the culture broths was checked by means of taking samples from the broth, which were extracted with ethyl acetate, concentrated in a vacuum and resuspended in methanol before analyzing them by means of UPLC. Finally, the culture broth was subjected to the process washing, extraction and purification as indicated in Example 2.

### EXAMPLE 5

### Producing the compound of formula (I) wherein R¹ is 2,2-dimethylpropionyl

Flasks containing 50 mL of M2 medium were inoculated (10% v/v) with inocula grown in MEB medium as indicated in Example 3 and they were incubated at 28°C, 250 rpm for 48 hours. After that time, sodium 2,2-dimethylpropionate (0.1%) (w/v) was added to the cultures maintaining the same incubation conditions as indicated in Example 3 for another 72-96 hours.

The presence of the compound of formula (I) wherein R¹ is 2,2-dimethylpropionyl, [(1*S*,3*R*,7*R*,8*S*,8*aR*)-8-[2-[(2*R*,4R)-4-hydroxy-6-oxo-oxan-2-yl]ethyl]-3,7-dimethyl-1,2,3,7,8,8*a-*hexahydro-naphthalen-1-yl]-2,2-dimethylpropionate in the culture broths was checked by means of taking samples from the broth, which were extracted with ethyl acetate, concentrated in a vacuum and resuspended in methanol before analyzing them by means of UPLC. Finally, the culture broth was subjected to the process of washing, extraction and purification indicated in Example 2.

## Claims

1. A process for obtaining a monacolin J derivative of formula (I) 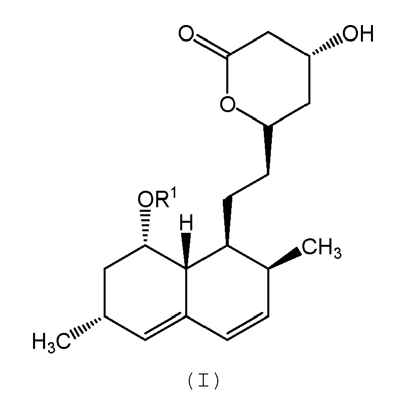 wherein R¹ is COR², wherein R² is selected from C1-C15 alkyl, C3-C15 cycloalkyl, C2-C15 alkenyl, C2-C15 alkynyl, aryl and heterocyclyl;
which comprises the steps of:
a) producing monacolin J by fermentation from a monacolin J-producing microorganism; and
b) acylating the hydroxyl group present in the C8 position of the monacolin J obtained in step a) by means of adding a suitable acylating agent to the fermentation medium to obtain the desired monacolin J derivative of formula (I)

2. The process according to claim 1, wherein said monacolin J-producing microorganism is a microorganism capable of producing and accumulating monacolin J at a concentration equal to or greater than 50 mg/L.

3. The process according to claim 1, wherein said monacolin J-producing microorganism is a microorganism belonging to a genus selected from *Aspergillus, Monascus, Penicillium* and *Neosartorya.*

4. The process according to claim 1, wherein said monacolin J-producing microorganism is *N. stramenia.*

5. The process according to claim 1, wherein said monacolin J-producing microorganism is the strain of *Neosartorya stramenia* CECT 20472 or a mutant of said microorganism that maintains the capacity for producing and accumulating monacolin J at a concentration equal to or greater than 50 mg/L.

6. The process according to claim 1, wherein said acylating agent is a compound of formula (II)
R²COOH (II)
wherein R² has the meaning previously indicated in relation to formula (I); or
a derivative thereof selected from a halide, an ester, an amide, an anhydride or a salt of said carboxylic acid of formula (II).

7. The process according to claim 1, wherein said acylating agent is selected from sodium propionate, sodium 2,2-dimethylpropionate, sodium 2,2-dimethylbutyrate and sodium 2-methylbutyrate.

8. The process according to claim 1, wherein said monacolin J derivative of formula (I) is a compound of formula (I) wherein R¹ is selected from propionyl, 2,2-dimethylpropionyl, 2-methylbutyryl (lovastatin) and 2,2-dimethylbutyryl (simvastatin).

9. The process according to claim 1 which further comprises isolating, and, optionally, purifying the monacolin J derivative of formula (I) obtained.

10. A microorganism of the genus *Neosartorya* which has the capacity for producing and accumulating monacolin J at a concentration equal to or greater than 50 mg/L.

11. The microorganism according to claim 1, **characterized in that** it is a microorganism of the species *Neosartorya stramenia* deposited in the Spanish Type Culture Collection (CECT) with accession number CECT 20472 which has the capacity for producing and accumulating monacolin J at a concentration equal to or greater than 50 mg/L, or a mutant of said microorganism that maintains the capacity for producing and accumulating monacolin J at a concentration equal to or greater than 50 mg/L.

12. A biologically pure culture of a microorganism according to claim 10 or 11.

13. Use of a microorganism according to any of claims 10 or 11 to produce monacolin J or a monacolin J derivative of formula (I) according to claim 1.

14. A process for identifying a monacolin J-producing microorganism which comprises:
a) incubating a culture of a microorganism in a plate inoculated with a culture of *Candida albicans* under conditions which allow the growth of said strain and of *C. albicans;*
b) analyzing the existence of antifungal activity associated with said microorganism;
c) if said microorganism does not show antifungal activity or shows low antifungal activity, collecting a sample from the culture of said microorganism and analyzing it to detect and/or quantify monacolin J in said sample; and
d) if said analysis shows the presence of monacolin J, identifying said microorganism as a monacolin J-producing microorganism.

15. The process according to claim 14, wherein the existence of fungicidal activity associated with said microorganism is analyzed by means of the formation of *C. albicans* growth inhibition halos.

16. The process according to claim 14 or 15 which further comprises using a positive control for antifungal activity.

17. The process according to claim 16 which comprises selecting the microorganisms that show *C. albicans* growth inhibition halos with a size smaller than that produced by said control.

18. The process according to any of claims 14 to 17, wherein the identified monacolin J-producing microorganism is a microorganism capable of producing and accumulating monacolin J at a concentration equal to or greater than 50 mg/L.


SEQUENCE LISTING

<110>  Neuron Biopharma, S.A.

<120>  Biosynthesis of monacolin J derivatives

<130>  P4178PC00

<150>  ES P200802971
<151>  2008-10-15

<160>  6

<170>  PatentIn version 3.5

<210>  1
<211>  583
<212>  DNA
<213>  Neosartorya stramenia

<400>  1
ttacgccagc gtccgagccg aagcgcgttc ctcggtccgg gcaggccgca ttgcaccctc      60

ggctataaga caccccgagg ggtgatacat tccgagggcc tttgaccggc cgcccaaacc     120

gacgctggcc cgcccacggg gaagtacacc ggcacgaatg ccggctgaac cccgcgggcg     180

agtctggtcg caaacgcttc cctttcaaca atttcacgtg ctgtttaact ctcttttcaa     240

agtgcttttc atctttcgat cactctactt gtgcgctatc ggtctccggc cagtatttag     300

ctttagatga aatttaccac ccatttagag ctgcattccc aaacaactcg actcgtcgaa     360

ggagcttcac acggacgcag acaccccgtc ccagacggga ttctcaccct ctatgacggc     420

ccgttccagg gcacttagac gggggctgca cccgaagcat cctctgcaaa ttacaacgcg     480

gaccccgagg gggccagctt tcaaatttga gctcttgccg cttcactcgc cgttactgag     540

gcaatccctg ttggtttctt ttcctccgct tattgatatg caa                       583


<210>  2
<211>  595
<212>  DNA
<213>  Neosartorya stramenia

<400>  2
gtgaccttga ggaaggttgg gccaaaggtt tgacggaaca ccttttaatt atttttacag      60

tgagtcaaat tttgaattaa tcttcaaaac tttcaacaac ggatctcttg gttctcgcat     120

cgatgaagaa cgcagcgaaa tgcgataagt aatatgaatt gcagattttc gtgaatcatc     180

gaatctttga acgcacattg cgccctctgg tattccagag ggcatgcctg tttgagcgtc     240

atttctctct caaacccccg ggtttggtat tgagtgatac tcttagtcgg actaggcgtt     300

tgcttgaaaa gtattggcat gggtagtact agatagtgct gtcgacctct caatgtatta     360

ggtttatcca actcgaagaa tggtgtggcg ggatatttct ggtattgttg gcccggcctt     420

acaacaacca aacaagtttg acctcaaatc aggtaggaat acccgctgaa cttaagcata     480

tcaataagcg gagggaagga ccgttacagt attcttttgc cagcgcttaa ctgcgcggcg     540

aaaaacctta cacacagtgt ctttttgata cagaactctt gctttggttt ggcct          595

<210>  3
<211>  24
<212>  DNA
<213>  Artificial Sequence

<220>
<223>  Oligonucleotide primer to amplify the D1/D2 region of
       28S subunit of the rDNA of Neosartorya stramenia

<400>  3
gcatatcaat aagcggagga aaag                                              24

<210>  4
<211>  19
<212>  DNA
<213>  Artificial Sequence

<220>
<223>  Oligonucleotide primer to amplify the D1/D2 region of
       28S subunit of the rDNA of Neosartorya stramenia

<400>  4
ggtccgtgtt tcaagacgg                                                    19

<210>  5
<211>  19
<212>  DNA
<213>  Artificial Sequence

<220>
<223>  Oligonucleotide primer to amplify the entire ITS region
       located between the 18S and 28S subunits of Neosartorya stramenia

<400>  5
tccgtaggtg aacctgcgg                                                    19

<210>  6
<211>  20
<212>  DNA
<213>  Artificial Sequence

<220>
<223>  Oligonucleotide primer to amplify the entire ITS region
       located between the 18S and 28S subunits of Neosartorya stramenia

<400>  6
tcctccgctt attgatatgc                                                   20